# EUROPÄISCHE PATENTSCHRIFT

(11) **EP 1 692 998 B1**
(45) Veröffentlichungstag und Bekanntmachung des Hinweises auf die Patenterteilung: **17.08.2011**
(21) Anmeldenummer: 05028292.0
(22) Anmeldetag: 23.12.2005
(51) Int. Cl.: A61B 3/16

(54) **Ophthalmisches Analysesystem zur Messung des intraokularen Drucks**
Ophthalmological analysis system for the measurement of the intraocular pressure
Système d'analyse ophthalmologique permettant la mesure de la pression intraoculaire

(30) Priorität: 16.02.2005 DE 202005002562 U
(43) Veröffentlichungstag der Anmeldung: 23.08.2006
(73) Patentinhaber: Oculus Optikgeräte GmbH, 35582 Wetzlar-Dutenhofen (DE)
(72) Erfinder: Köst, Gert, 30171 Hannover (DE)
(74) Vertreter: von den Steinen, Axel

(56) Entgegenhaltungen:
- WO-A-03/096888
- US-A- 5 002 056
- US-A- 5 474 066
- US-A- 6 053 867
- US-A- 6 120 444

## Beschreibung

Die Erfindung betrifft ein ophthalmisches Analysesystem zur Messung des intraocularen Drucks im Auge (Augeninnendruck) nach dem Oberbegriff des Anspruchs 1.

Durch einen erhöhten Augeninnendruck können schwerwiegende Gesundheitsbeeinträchtigungen ausgelöst werden. Insbesondere kann der Sehnerv durch den erhöhten Augeninnendruck geschädigt werden, wodurch der sogenannte grüne Star (Glaukom) mit Einschränkungen des Gesichtsfeldes verursacht wird.

Zur Überprüfung des intraocularen Drucks sind drei Grundprinzipien, nämlich die Impressionstonometrie, die Applanationstonometrie und die Non-Kontakt-Tonometrie, bekannt. Beim Impressionstonometer misst man die Tiefe der Eindellbarkeit der Hornhaut, hervorgerufen durch einen mit einem bekannten Gewicht belasteten Metallstempel. Bei dem selben Gewicht verhält sich die Eindellbarkeit umgekehrt proportional zum Augeninnendruck, das heißt, die Eindellbarkeit ist um so größer, je niedriger der Augeninnendruck ist und umgekehrt. Nachteilig an der Impressionstonometrie ist es, dass das Aufsetzen des Tonometers und das Eindrücken des Metallstempels den Augeninnendruck zusätzlich erhöht, so dass der gemessene Druck nicht exakt dem tatsächlichen Augeninnendruck entspricht. Weiterhin ist das Aufsetzen des Stempels auf die Hornhaut des Patientenauges für die Patienten relativ belastend.

Weiterhin sind zur Messung des Augeninnendrucks sogenannte Applanationstonometer bekannt, deren Messung auf der Anwendung des Applanationsprinzips beruht. Das Applanationsprinzip geht von dem Gesetz von Ingbert aus, das besagt, dass der Druck in einem mit Flüssigkeit angefüllten kugelförmigen Behälter dem Gegendruck entspricht, der eine bestimmte Oberfläche dieser Kugel abplattet. Die Augeninnendruckmessung auf Grundlage dieses Gesetzes ist auf zwei verschiedene Arten möglich. Nach der ersten Alternative kann ein Tonometer mit konstantem Gewicht verwendet und die abgeplattete Oberfläche gemessen werden. Nach der alternativen Messmethode wird die Kraft festgestellt, die erforderlich ist, um eine bekannte Oberfläche konstanter Größe zu applanieren. Bekannt ist ein Applanationstonometer nach Perkins, das aus einem Kunststoffzylinder besteht, dessen unteres planares Ende mit seiner Gradeinteilung versehen ist. Am oberen Ende befindet sich eine Lupe. Nach Einträufeln einer fluoreszierenden Flüssigkeit in den Bindehautsack lässt sich der Durchmesser der applanierten Hornhautfläche durch optisches Ablesen an der Gradskala bestimmen. Die Bestimmung des Augeninnendrucks erfolgt hier mittels einer konstanten Kraft.

Weiterhin bekannt ist ein Applanationstonometer das auf Grundlage einer applanierten Oberfläche konstanter Größe arbeitet. Die Hornhaut wird hierbei mit Hilfe der viereckigen Basis eines Glasprismas abgeplattet. Der Augeninnendruck wird gemessen, indem der Druck des Prismas auf das Auge so lange verstärkt wird, bis der abgeplattete kreisförmige Hornhautbezirk mit den vier Seiten der Prismabasis niveaugleich ist. Nachteilig bei den Applanationstonometern ist es wiederum, dass durch die Verformung der Hornhaut mittels eines Betätigungselements eine erhebliche Belastung für den Patienten entsteht.

Zur Vermeidung dieser durch die Berührung mit einem Verformungswerkzeug entstehenden Belastung wurden sogenannte Non-Kontakt-Tonometer entwickelt. Bei diesen Non-Kontakt-Tonometern sind Betätigungseinrichtungen zur Verformung der Hornhaut vorgesehen, mit denen die Hornhaut berührungsfrei verformt wird. Dazu kann beispielsweise ein Druckluftimpuls erzeugt und auf die Hornhaut gerichtet werden. Bei bekannten Non-Kontakt-Tonometern werden Luftstöße auf das Auge in Richtung der optischen Achse gerichtet, wodurch die Hornhaut mehr und mehr abgeflacht und schließlich eingedellt wird. Zur Messung der Verformung der Hornhaut wird ein schräg einfallendes Bündel paralleler Lichtstrahlen auf die Hornhaut gerichtet und das von der Hornhaut reflektierte Licht als Messsignal gemessen. Dazu kann das reflektierte Licht beispielsweise mit einem Lichtsensor aufgefangen werden, wobei sich die vom Lichtsensor gemessene Lichtintensität in Abhängigkeit von der durch den Luftstrom verursachten Applanation der Hornhaut verändert.

Die US 5,474,066 offenbart eine Tonometeranordung mit einer pachymetrischen Vorrichtung zur Messung der Korneadicke, die zur Korrektur des Wertes des Augeninnendrucks verwendet wird. Zur Messung der Korneadicke wird ein Bereich der Kornea beleuchtet und auf einem Detektorarray abgebildet.

Bei der US 6,053,867 wird mit von der Kornea reflektierten Lichtstrahlen ein Spaltbild erzeugt, dessen Lichtidentitätsverteilung ausgewertet wird, um die Lage einer Augeninnendruck-Messvorrichtung zu korrigieren.

Bei der US 5,002,056 wird der Grad der Verformung des Auges erfasst, in dem die Änderungen der optischen Menge des an der Hornhaut reflektierten Lichts gemessen wird.

Die WO 03/096888 offenbart einen berührungslos arbeitenden Tonometer, der zwei Videokameras aufweist, die zur Ermittlung des richtigen Messabstandes zwischen Tonometer und Auge verwendet werden.

Die US 6,120,444 zeigt ein ophthalmisches Analysesystem welches vermittels eines Spaltprojektors einen Lichtspalt auf eine Hornhaut eines Auges projiziert und mit einer Aufnahmeeinrichtung bzw. Scheimpflugkamera ein Schnittbild aufnimmt. Vor Erzeugung eines definierten Luftstoßes auf die Hornhaut wird ein erstes Schnittbild der Hornhaut aufgenommen. Während des Aufbringens des Luftstoßes wird nach einem definierten Zeitabschnitt ein zweites Schnittbild der Hornhaut aufgenommen. Weiter werden die beiden Schnittbilder überlagert und mit in einem Speicher einer Analyseeinrichtung vorhandenen Schnittbildern verglichen, wobei aus einer Verformung der Hornhaut ein intraokularer Druck ableitbar ist.

Ausgehend von diesem Stand der Technik ist es die Aufgabe der vorliegenden Erfindung ein neues ophthalmisches Analysesystem zur Messung des intraocularen Drucks mit einer alternativen Lösung zur Bestimmung der Elastizität der Hornhaut des Auges vorzuschlagen.

Diese Aufgabe wird durch ein Analysesystem nach der Lehre des Anspruchs 1 gelöst.

Vorteilhafte Ausführungsformen der Erfindung sind Gegenstand der

Unteransprüche.

Das erfindungsgemäße Analysesystem beruht auf dem Grundgedanken, dass vor und/oder während und/oder nach der Verformung der Hornhaut Schnittbilder der Hornhaut aufgenommen werden, die den Zustand der Hornhaut in einer Schnittebene zeigen. Diese Schnittbilder werden in der Analyseeinrichtung mittels geeigneter Bildverarbeitungsverfahren analysiert und geben zusätzliche Informationen über den Zustand der

Hornhaut, die bei der Ableitung des Augeninnendruckes berücksichtigt werden können.

Großen Einfluss auf die Messwerte und damit auf das Messergebnis des Augeninnendruckes hat die Dicke und die Elastizität der Hornhaut, da die Hornhaut als elastisch verformbare Membran der von der Betätigungseinrichtung aufgebrachten Verformungskraft eine Gegenkraft entgegensetzt, die vom Augeninnendruck selbst nicht abhängt und somit die Messung des Augeninnendruckes verfälschen kann. Es ist deshalb besonders vorteilhaft aus den Schnittbildern der Hornhaut die Lichtstreuung der Hornhaut zu bestimmen. Die Lichtstreuung der Hornhaut steht mit dem Kennwert der Elastizität der Hornhaut in einem bestimmten Zusammenhang, so dass aus der Lichtstreuung auf die Elastizität der Hornhaut zurückgeschlossen werden kann. Weiter kann aus den Schnittbildern der Hornhaut die Dicke der Hornhaut abgeleitet werden. Unter Berücksichtigung der bekannten Elastizitätskennwerte der Hornhaut, kann aus der Dicke der Hornhaut dann die von der Hornhaut bei der elastischen Verformung aufgebrachte Gegenkraft abgeschätzt und bei der Ableitung des Augeninnendruckes als Einflussfaktor berücksichtigt werden.

Alternativ oder additiv zur Bestimmung der Dicke der Hornhaut als Einflussfaktor kann aus den Schnittbildern der Hornhaut auch die Krümmung der Hornhaut abgeleitet werden. Auch die Krümmung der Hornhaut hat Einfluss auf die Messergebnisse und sollte deshalb bei der Ableitung des Augeninnendrucks Berücksichtigung finden.

Mit welcher Messmethode der Augeninnendruck selbst gemessen und im Analysesystem abgeleitet wird, ist grundsätzlich beliebig. Beispielsweise können dazu die bekannten Reflektionslichtmethoden eingesetzt werden, wobei die erfindungsgemäß aufgenommenen Schnittbilder dann beispielsweise nur zur Korrektur des Einflusses herangezogen wird, der durch die elastische Verformung der Hornhaut entsteht. Besonders vorteilhaft ist des jedoch, wenn auch der Augeninnendruck aus den Schnittbildaufnahmen der verformten Hornhaut abgeleitet wird. Diese Schnittbildaufnahmen stellen nämlich die durch Betätigungseinrichtung verursachte Verformung der Hornhaut außerordentlich exakt dar und enthalten damit die für die Ableitung des Augeninnendruckes erforderlichen Bildinformationen. So können beispielsweise während der Verformung der Hornhaut eine Vielzahl von Schnittbildern als Bildserie hintereinander aufgenommen werden, so dass in der dann anschließenden Bildanalyse das Schnittbild mit der größten Hornhautverformung extrahierbar ist. Aus diesem Bild der Hornhaut mit der größten Verformung kann dann unter Berücksichtigung der Dicke der Hornhaut der Augeninnendruck einfach abgeleitet werden.

In welcher Weise die Schnittbildaufnahmen der Hornhaut gerätetechnisch ermöglicht werden, ist grundsätzlich beliebig. Besonders vorteilhaft ist es, wenn das Beobachtungssystem einen Spaltprojektor umfasst, mit dem ein Lichtspalt auf die Hornhaut projiziert werden kann. Derartige Spaltprojektoren sind aus der Augenheilkunde bekannt. Das notwendige Beleuchtungsprinzip des Spaltprojektors beruht dabei darauf, dass die brechenden Medien der Augenvorkammer nicht glasklar sind, sondern an ihnen, insbesondere im kurzwelligen Anteil des sichtbaren Lichtes eine deutliche Streuung erfolgt. Dieses führt dazu, dass ein gebündelter Lichtstrahl, das heißt vorliegend der projizierte Lichtspalt, den man durch die optischen Medien des Auges schickt, bei seitlicher Betrachtung die Augenstrukturen und insbesondere die Hornhaut als Schnittbild sichtbar macht, da das Licht beim Durchtritt durch die unterschiedlichen Materialien, insbesondere beim Durchtritt durch die Hornhaut, unterschiedlich stark gestreut wird. Der spaltförmige Lichtstrahl erzeugt dadurch eine Bildebene, die im Schnitt durch den Augenkörper verläuft, so dass die mit dem Beobachtungssystem aufzunehmenden Schnittbilder gerade in dieser durch den Lichtspalt definierten Bildebene liegen.

Um die durch den Lichtspalt beleuchteten Schnittbilder aufnehmen zu können, sollte das Beobachtungssystem eine Aufnahmeeinrichtung umfassen, die derart angeordnet ist, dass die vom Spaltprojektor beleuchtete Bildebene zumindest teilweise aufnehmbar ist.

Zur Erhöhung der Bildqualität kann zwischen Hornhaut und Aufnahmeeinrichtung zumindest ein Objektiv, das heißt eine Linsenanordnung angeordnet werden. Mit dieser Linsenanordnung wird die vom Spaltprojektor beleuchtete Bildebene der Hornhaut auf eine Aufnahmeebene in der Aufnahmeeinrichtung abgebildet.

Um eine große Tiefenschärfe in den Schnittbildern zu erhalten, sollte die Anordnung der vom Spaltprojektor beleuchteten Bildebene in der Hornhaut, der Hauptebene des Linsensystems zwischen der Hornhaut und der Aufnahmeeinrichtung (Objektivebene) und die Aufnahmeebene der Aufnahmeeinrichtung der Scheimpflugbedingung entsprechen. Diese von dem Photographen Scheimpflug entwickelte Regel schreibt vor, dass die Bildebene, die Objektivebene und die Aufnahmeebene derart winkelig angeordnet sind, dass sie sich in einer gemeinsamen Achse schneiden. Durch das Kippen der Aufnahmeebene relativ zur Objektivebene kann die Bildebene in eine beliebige räumliche Lage gebracht werden, wobei in der Schärfenzone Bildpunkte erfasst werden, die bei senkrechter Bildebene ansonsten nicht zugleich scharf abgebildet werden können.

Zur Berührungsfreien Verformung der Hornhaut ist es besonders vorteilhaft, wenn mit der Betätigungseinrichtung ein Strömungsimpuls eines gasförmigen Mediums, insbesondere Luft auf die Hornhautoberfläche aufgebracht werden kann. Die Belastung des Patienten durch solch einen Luftstrahl ist relativ sehr gering und wird aufgrund seiner Kürze als nicht sehr belastend wahrgenommen.

Konstruktiv kann die Betätigungseinrichtung dadurch realisiert werden, dass ein Druckkammer mit einer auf das zu untersuchende Auge gerichteten Düsenöffnung vorgesehen ist. Durch kurzfristige Erhöhung des Drucks in der Druckkammer strömt das in der Druckkammer befindliche Gas durch die Düsenöffnung aus und bildet auf diese Weise den gewünschten Strömungsimpuls auf der Augenoberfläche. Die Erhöhung des Drucks in der Druckkammer kann beispielsweise dadurch entstehen, dass ein mechanisch angetriebener Stempel in einer Zylinderöffnung der Druckkammer bewegt wird.

Um die Verformung der Hornhaut mit der Stärke des Strömungsimpulses in Korrelation setzten zu können, sollte in oder an der Druckkammer ein Sensor, beispielsweise ein Drucksensor vorgesehen sein, mit dem die Stärke des Strömungsimpulses direkt oder indirekt gemessen werden kann. Wird beispielsweise mit einem Drucksensor die Erhöhung des Innendrucks in der Druckkammer gemessen, kann aus diesem Druckverlauf bei bekanntem Durchmesser der Düsenöffnung die Stärke des Strömungsimpulses indirekt abgeleitet werden. Dieser Messwert kann dann ebenfalls an die Analyseeinrichtung weitergeleitet werden und dort bei den Berechnungen der anderen Messparameter Berücksichtigung finden.

Um eine möglichst genaue Messung der Hornhautverformung zu ermöglichen, sollte der Strahlengang des Spaltprojektors beim Auftreffen auf die Hornhaut koaxial zur Längsachse des Strömungsimpulses verlaufen.

Dies kann insbesondere dadurch realisiert werden, dass der Strahlengang des Spaltprojektors durch die Betätigungseinrichtung verläuft.

An den Durchtrittspunkten des Strahlengangs durch die Betätigungseinrichtung sind dabei entweder Ausnehmungen, wie insbesondere eine durch die Düsenöffnung gebildet wird, oder transparente Materialien vorzusehen, die vom Licht des Spaltprojektors durchlaufen werden können.

Alternativ dazu kann auch eine Umlenkoptik vorgesehen sein, mit der der Strahlengang des Spaltprojektors an der Betätigungseinrichtung und der Düsenöffnung vorbeigeführt wird.

Welche Art von Aufnahmeeinrichtung zur Aufnahme der Schnittbilder eingesetzt wird, ist grundsätzlich beliebig. Besonders vorteilhaft ist es dabei, wenn es sich um eine Hochgeschwindigkeitsaufnahmeeinrichtung handelt, mit der in rascher Bildfolge Schnittbilder aufgenommen werden können, so dass während der Verformung der Hornhaut die Aufnahme mehrerer Schnittbilder möglich ist. Durch Analyse dieser Bilderfolge, die die Hornhaut vom Anfang der Verformung über den Höhepunkt der Verformung bis zum Ende der Verformung zeigt, können die gewünschten Messparameter sehr genau abgeleitet werden.

Besonders bevorzugt ist es dabei, wenn als Aufnahmeeinrichtung ein Videosensor vorgesehen ist, der die Bilddaten der Schnittbilder in Form eines Videosignals weitergibt. Das Videosignal sollte dabei vorzugsweise in digitalem Format erzeugt oder aus einem analogem Format in ein digitales Format umgewandelt werden, da digitale Videosignal in bekannten Datenverarbeitungsanlagen sehr gut durch bekannte Bildverarbeitungssysteme analysiert werden können.

Insbesondere können als Videosensoren CCD-Chips oder CMOS-Chips Verwendung finden, da diese hochauflösende Aufnahmen ermöglichen und zugleich kostengünstig verfügbar sind. Bei den erfindungsgemäßen Schnittbildern der Hornhaut muss es sich nicht zwingend um Vollbilder handeln, in denen der aufgenommene Hornhautabschnitt vollständig bildlich dargestellt ist. Vielmehr können auch Zeilenkameras oder auch mehrere nebeneinander angeordnet Zeilenkameras als Videosensor Verwendung finden. Die Zeilenkameras sind dabei in der Aufnahmeebene so anzuordnen, dass die Abbildung der Hornhaut derart auf die Zeilenkamera abgebildet wird, dass eine Verformung der Hornhaut durch die Zeilenkameras erkannt werden kann.

Alternativ zur Verwendung von Zeilenkameras können selbstverständlich auch Flächenkameras, beispielsweise flächige CCD-Chips oder CMOS-Chips als Videosensor eingesetzt werden.

Um den behandelnden Arzt die Ausrichtung des zu untersuchenden Auges am Analysesystem zu vereinfachen, kann das Analysesystem eine Einrichtkamera aufweisen.

Eine Ausführungsform der Erfindung ist in den Zeichnungen schematisch dargestellt und wird nachfolgend beispielhaft erläutert.

Es zeigen:
- **Fig. 1:**: Den Aufbau eines erfindungsgemäßen Analysesystems zur Messung des intraocularen Drucks;
- **Fig. 2:**: Die Schnittbildebene eines schematisch dargestellten Auges mit unverformter Hornhaut;
- **Fig. 3:**: Die Schnittbildebene des Auges gemäß Fig. 2 mit verformter Hornhaut und Anordnung des Bildbereiches einer Flächenkamera;
- **Fig. 4:**: Die Schnittbildebene gemäß Fig. 2 mit verformter Hornhaut und Anordnung des Bildbereiches mit mehreren Zeilenkameras.

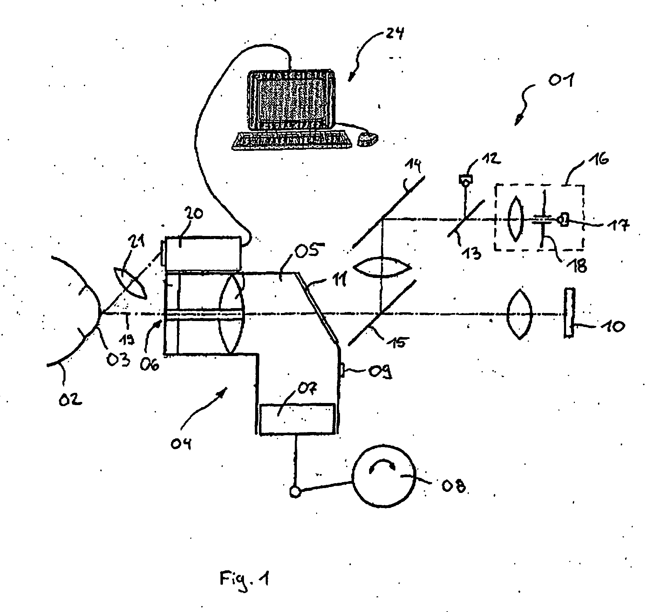

Das in **Fig. 1** schematisch dargestellte Analysesystem 01 dient zur Untersuchung eines schematisch dargestellten Auges 02. Insbesondere kann mit dem Analysesystem 01 der Augeninnendruck des Auges 02 und die Dicke der Hornhaut 03 gemessen werden.

Um den Augeninnendruck des Auges 02 feststellen zu können, muss die Hornhaut 03 geringfügig verformt werden. Dazu dient eine Betätigungseinrichtung 04. Die Betätigungsvorrichtung 04 umfasst eine geschlossene Druckkammer 05, die an ihrer zum Auge 02 weisenden Seite eine Düsenöffnung 06 aufweist. Zur Erhöhung des Innendrucks in der Druckkammer 05 dient ein Kolben 07, der durch Antrieb einer Antriebwelle 08 in der Druckkammer 05 auf und ab bewegt werden kann. Wird der Kolben 07 mit einer schnellen Stellbewegung ins innere der Druckkammer 05 eingefahren, so wird die Luft in der Druckkammer 05 durch die Düsenöffnung 06 nach außen verdrängt und dadurch ein Strömungsimpuls erzeugt, der auf die Hornhaut 03 auftrifft und diese berührungslos verformt. Um die Stärke des Druckimpulses messen zu können, ist an der Druckkammer 05 ein Drucksensor 09 vorgesehen, mit dem der Anstieg des Innendrucks in der Druckkammer 05 gemessen werden kann. Aus diesen Druckwerten kann dann die Stärke des Strömungsimpulses, der durch die Düsenöffnung 06 auf die Hornhaut 03 auftrifft, abgeleitet werden.

Um das Auge 02 lagerichtig zur Analyseeinrichtung 01 ausrichten zu können, dient eine Einrichtkamera 10. Mit der Einrichtkamera 10 kann das Auge 02 durch eine transparente Abdeckung 11 der Druckkammer 05 und durch die Düsenöffnung 06 hindurch anvisiert werden so dass der behandelnde Arzt die lagerichtige Ausrichtung des Auges 02 zur Analyseeinrichtung 01 beurteilen kann. Um das Auge 02 in der gewünschten Lage während der Untersuchung fixieren zu können, ist ein Fixierlicht 12 vorgesehen, dessen sichtbares Licht mittels der Spiegel 13, 14 und 15 auf das Auge 02 durch die Betätigungseinrichtung 04 hindurch gelenkt wird. Die Spiegel 13 und 15 sind dabei halbdurchlässig ausgebildet.

Weiter ist in dem Analysesystem 01 ein Spaltprojektor 16 vorgesehen, mit dem ein Lichtspalt auf die Hornhaut 03 projiziert werden kann. Im Spaltprojektor 16 wird das Licht mittels eines Leuchtmittels 17 erzeugt und mittels einer Spaltblende 18 zu einem Lichtspalt geformt. Als Leuchtmittel im Spaltprojektor 16 können dabei Xenonhochdrucklampen oder geeignete Leuchtdioden Verwendung finden.

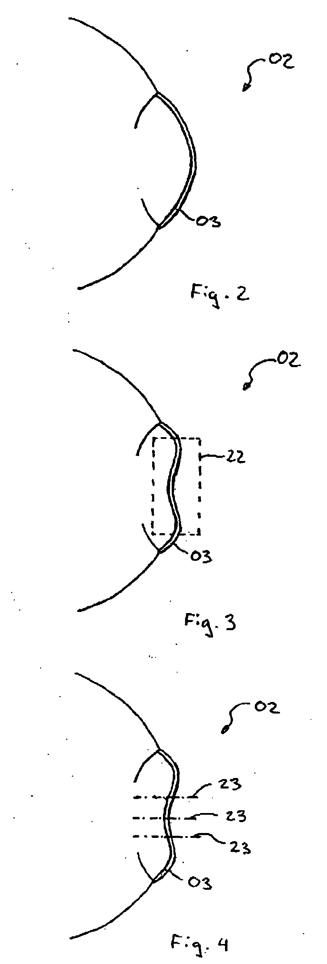

Mit dem im Spaltprojektor 16 erzeugten Lichtspalt wird das Auge 02 in einer Schnittebene beleuchtet, die entlang der optischen Hauptachse 19 senkrecht zur Bildebene von Fig. 1 verläuft. Derartige Schnittbilder sind schematisch in **Fig. 2** bis **Fig. 4** dargestellt.

Zur Beobachtung und Aufnahme vor, während und nach der Verformung dient eine Aufnahmeeinrichtung 20, mit der die durch den Lichtspalt beleuchteten Schnittebenen unter einem Winkel beobachtet werden können. Zwischen der Aufnahmeeinrichtung 20 und dem Auge 02 ist dabei ein Objektiv 21 derart angeordnet, dass von den durch den Lichtstrahl beleuchteten Schnittebenen der Hornhaut Scheimpflugaufnahmen gemacht werden können. In der Aufnahmeeinrichtung 20 dient ein CCD-Chip oder CMOS-Chip als Videosensor, dessen Bilddaten an eine Analyseeinrichtung 24, die softwaremäßig auf einem Industrie - PC installiert ist, weitergeleitet wird.

In **Fig. 2** bis **Fig. 3** ist das Auge 02 in der durch den Spaltprojektor 16 beleuchteten Schnittbildebene schematisch dargestellt. **Fig. 2** zeigt dabei das Auge 02 mit unverformter Hornhaut 03. Die Dicke der Hornhaut 03 kann durch Bilddatenverarbeitung der mit der Aufnahmeeinrichtung 20 aufgenommenen Bilder abgeleitet und bei der Berechnung des Augeninnendruckes berücksichtigt werden.

**Fig. 3** zeigt den strichliniert umrandeten Bereich, der mit dem in der Aufnahmeeinrichtung 20 eingebauten CCD- oder CMOS-Videosensor aufgenommen werden kann. Der Bildbereich umfasst eine rechteckige Fläche, der die Hornhaut 03 in ihrem Zentrum umfasst. Während der Verformung der Hornhaut 03 werden mit der Aufnahmeeinrichtung mehrere Schnittbilder aufgenommen und anschließend durch Bilddatenverarbeitung aus der Bildsequenz der verformten Hornhaut 03 unter Berücksichtigung der Dicke der Hornhaut 03 und den Messdaten des Drucksensors 09 der Augeninnendruck abgeleitet.

Alternativ zu einer Flächenkamera können in der Aufnahmeeinrichtung 20 auch eine oder mehrere Zeilenkameras eingesetzt werden. Der Bildbereich 23 dieser Reihenkameras ist in **Fig. 4** schematisch dargestellt.

## Patentansprüche

1. Ophthalmisches Analysesystem (01) zur Messung des intraokularen Drucks in einem Auge (02) mit
a) einer Betätigungseinrichtung (04) zur berührungsfreien Verformung der Hornhaut (03),
b) einem Beobachtungssystem (20, 21) mit dem die Verformung der Hornhaut beobachtet und aufgenommen werden kann,
c) einer Analyseeinrichtung (24) mit der aus den Bildinformationen des Beobachtungssystems (20, 21) der intraokulare Druck abgeleitet wird , wobei mit dem Beobachtungssystem (20, 21) Schnittbilder von zumindest Teilen der unverformten und/oder verformten Hornhaut (03) aufgenommen werde, **dadurch gekennzeichnet,**
**dass** die Analyseeinrichtung (24) derart eingereichtet ist, dass aus den Schnittbildern der Hornhaut (03) die Lichtstreuung der Hornhaut (03) bestimmt wird, wobei die Lichtstreuung der Hornhaut (03) als ein Maß für die Elastizität der Hornhaut (03) abgeleitet wird, und die aus der Lichtstreuung abgeleitete Elastizität der Hornhaut (03) als Einflussfaktor bei der Ableitung des intraokularen Drucks berücksichtigt wird.

2. Analysesystem nach Anspruch 1,
**dadurch gekennzeichnet,**
**dass** in der Analyseeinrichtung (24) aus den Schnittbildern der Hornhaut (03) die Dicke der Hornhaut (03) abgeleitet wird.

3. Analysesystem nach Anspruch 1,
**dadurch gekennzeichnet,**
**dass** in der Analyseeinrichtung (24) aus den Schnittbildern der Hornhaut (03) die Krümmung der Hornhaut (03) abgeleitet wird.

4. Analysesystem nach einem der Ansprüche 2 bis 3,
**dadurch gekennzeichnet,**
**dass** bei der Ableitung des intraokularen Drucks die Dicke der Hornhaut (03) und/oder die Krümmung der Hornhaut (03) als Einflussfaktor_berücksichtigt wird.

5. Analysesystem nach einem der Ansprüche 1 bis 4,
**dadurch gekennzeichnet,**
**dass** in der Analyseeinrichtung (24) aus den Schnittbildern der verformten Hornhaut (03), insbesondere aus einer Serie von Schnittbildern der Hornhaut (03), der intraokularen Druck im Auge (02) abgeleitet wird.

6. Analysesystem nach einem der Ansprüche 1 bis 5,
**dadurch gekennzeichnet,**
**dass** das Beobachtungssystem (20, 21) mit einem Spaltprojektor (16) zusammenwirkt, mit dem ein Lichtspalt auf die Hornhaut (03) projiziert wird, wobei die mit dem Beobachtungssystem (20, 21) aufzunehmenden Schnittbilder in einer vom Spaltprojektor beleuchteten Bildebene liegen.

7. Analysesystem nach Anspruch 6,
**dadurch gekennzeichnet,**
**dass** das Beobachtungssystem (20, 21) eine Aufnahmeeinrichtung (20) umfasst, mit der die Hornhaut (03) in der vom Spaltprojektor (16) beleuchteten Bildebene zumindest teilweise aufgenommen wird.

8. Analysesystem nach Anspruch 7,
**dadurch gekenntzeichnet**,
dass zwischen Hornhaut (03) und Aufnahmeeinrichtung (20) zumindest ein Objektiv (21) angeordnet ist, mit der die vom Spaltprojektor (16) beleuchtete Bildebene der Hornhaut (03) auf eine Aufnahmeebene in der Aufnahmeeinrichtung (20) abgebildet wird.

9. Analysesystem nach Anspruch 8,
**dadurch gekennzeichnet,**
**dass** die vom Spaltprojcktor (16) beleuchtete Bildebene der Hornhaut (03) und die Objektivebene des zwischen der Hornhaut (03) und der Aufnahmeeinrichtung (20) angeordneten Objektivs (21) und die Aufnahmeebene in der Aufnahmeeinrichtung (20) derart winkelig angeordnet sind, dass die Bildebene der Hornhaut (03) entsprechend der Scheimpflugbcdingung auf die Aufnahmeebene der Aufnahmeeinrichtung (20) abgebildet wird.

10. Analysesystem nach einem der Ansprüche 1 bis 9,
**dadurch gekennzeichnet,**
**dass** mit der Betätigungseinrichtung (04) zur Verformung der Hornhaut (03) ein Strömungsimpuls eines gasförmigen Mediums, insbesondere Luft, auf die Hornhautoberfläche aufgebracht wird.

11. Analysesystem nach Anspruch 10,
**dadurch gekennzeichnet,**
**dass** an der Betätigungseinrichtung (04) eine zumindest teilweise transparente Druckkammer (05) mit einer auf das zu untersuchende Auge gerichteten Düsenöffnung (06) vorgesehen ist, wobei durch Erhöhung des Drucks in der Druckkammer (05) ein auf das Auge gerichteter Strömungsimpuls erzeugt wird.

12. Analysesystem nach Anspruch 11,
**dadurch gekennzeichnet,**
**dass** in oder an der Druckkammer (05) ein Sensor (09) zur direkten oder indirekten Messung der Stärke des Strömungsimpulses vorgesehen ist.

13. Analysesystem nach einem der Ansprüche 10 bis 12,
**dadurch gekennzeichnet,**
**dass** der Strahlengang des vom Spaltprojektor (16) erzeugten Lichtspalts beim Auftreffen auf die Hornhaut (03) koaxial zur Längsachse des Strömungsimpulses des gasförmigen Mediums verläuft.

14. Analysesystem nach einem der Ansprüche 10 bis 13,
**dadurch gekennzeichnet,**
**dass** der Strahlengang des vom Spaltprojektor (16) erzeugten Lichtspalts durch die Betätigungseinrichtung (04) verläuft, wobei die Betätigungseinrichtung (04) an den Durchtrittspunkten des Strahlengangs Ausnehmungen (06) aufweist oder aus transparentem Material (11) hergestellt ist.

15. Analysesystem nach Anspruch 14,
**dadurch gekennzeichnet,**
**dass** der Strahlengang des vom Spaltprojektor (16) erzeugten Lichtspalts durch die Düsenöffnung (06) verläuft.

16. Analysesystem nach einem der Ansprüche 10 bis 13,
**dadurch gekennzeichnet,**
**dass** vor und/oder hinter der Düsenöffnung in der Druckkammer eine Umlenkoptik angeordnet ist, mit der der Strahlengang des Spaltprojektors an der Düsenöffnung vorbeigeführt werden kann.

17. Analysesystem nach einem der Ansprüche 1 bis 16,
**dadurch gekennzeichnet,**
**dass** die Aufnahmeeinrichtung (20) in der Art einer Hochgeschwindigkeitsaufnahmeeinrichtung ausgebildet ist, mit der während der Verformung der Hornhaut (03) mehrere Schnittbilder als Bildserie aufgenommen werden .

18. Analysesystem nach einem der Ansprüche 1 bis 17,
**dadurch gekennzeichnet,**
**dass** in der Aufnahmeeinrichtung (20) ein Videosensor vorgesehen ist, mit dem die Verformung der Hornhaut (03) beobachtet und aufgenommen werden kann, wobei der Videosensor die entsprechenden Bilddaten in Form eines Videosignal weitergibt.

19. Analysesystem nach Anspruch 18,
**dadurch gekennzeichnet,**
**dass** das Videosignal in einem digitalem Format erzeugt oder umgewandelt wird.

20. Analysesystem nach Anspruch 19,
**dadurch gekennzeichnet,**
**dass** der Videosensor in der Art eines CCD-Chips oder CMOS-Chips ausgebildet ist.

21. Analysesystem nach einem der Ansprüche 18 bis 20,
**dadurch gekennzeichnet,**
**dass** der Videosensor von zumindest einer Zeilenkamera gebildet wird.

22. Analysesystem nach Anspruch 21,
**dadurch gekennzeichnet,**
**dass** der Videosensor von mehreren parallel zueinander beabstandet angeordneten Zeilenkameras gebildet wird.

23. Analysesystem nach einem der Ansprüche 18 bis 20,
**dadurch gekennzeichnet,**
**dass** der Videosensor von einer Flächenkamera gebildet wird.

24. Analysesystem nach einem der Ansprüche 1 bis 23,
**dadurch gekennzeichnet,**
**dass** im Analysesystem (01) eine Einrichtkamera (10) zur lagerichtigen Aufrichtung des zu untersuchenden Auges (02) vorgesehen ist.

## Claims

1. Ophthalmic analysis system (01) for measuring the intraocular pressure in an eye (02) with
a) an actuating device (04) for contact-free deformation of the cornea (03),
b) an observation system (20, 21) with which the deformation of the cornea can be observed and recorded,
c) an analysis device (24) with which the intraocular pressure can be derived from the image information of the observation system (20, 21) wherein sectional images of at least parts of the undeformed and/or deformed cornea (03) are recorded using the observation system (20, 21),
**characterized in that**
the analysis device (24) is arranged such that the light scattering of the cornea (03) is determined from the sectional images of the cornea (03), wherein the light scattering of the cornea (03) is derived as a measure for the elasticity of the cornea (03), and the elasticity of the cornea (03) derived from the light scattering is taken into account as an influential factor in the derivation of the intraocular pressure.

2. Analysis system according to Claim 1,
**characterized in that**
in the analysis device (24) the thickness of the cornea (03) is derived from the sectional images of the cornea (03).

3. Analysis system according to Claim 1,
**characterized in that**
in the analysis device (24) the curvature of the cornea (03) is derived from the sectional images of the cornea (03).

4. Analysis system according to one of Claims 2 to 3,
**characterized in that**
the thickness of the cornea (03) and/or the curvature of the cornea (03) is taken into account as an influential factor in the derivation of the intraocular pressure.

5. Analysis system according to one of Claims 1 to 4,
**characterized in that**
the intraocular pressure in the eye (02) is derived in the analysis device (24) from the sectional images of the deformed cornea (03), in particular from a series of sectional images of the cornea (03)

6. Analysis system according to one of Claims 1 to 5,
**characterized in that**
the observation system (20, 21) cooperates with a slit projector (16), by which a light slit is projected onto the cornea (03), wherein the sectional images which are to be recorded with the observation system (20, 21) lie in an image plane which is illuminated by the slit projector.

7. Analysis system according to Claim 6,
**characterized in that**
the observation system (20, 21) comprises a recording device (20) by which the cornea (03) is at least partially recorded in the image plane which is illuminated by the slit projector (16).

8. Analysis system according to Claim 7,
**characterized in that**
between the cornea (03) and the recording device (20) at least one objective (21) is arranged, by which the image plane of the cornea (03), which is illuminated by the slit projector (16) is imaged onto a recording plane in the recording device (20).

9. Analysis system according to Claim 8,
**characterized in that**
the image plane of the cornea (03), which is illuminated by the slit projector (16), and the objective plane of the objective (21) which is arranged between the cornea (03) and the recording device (20), and the recording plane in the recording device (20) are arranged at an angle such that the image plane of the cornea (03) is imaged according to the Scheimpflug condition onto the recording plane of the recording device (20).

10. Analysis system according to one of Claims 1 to 9,
**characterized in that**
a flow pulse of a gaseous medium, in particular air, is applied onto the surface of the cornea using the actuating device (04) for deformation of the cornea (03).

11. Analysis system according to Claim 10,
**characterized in that**
an at least partially transparent pressure chamber (05) with a nozzle opening (06) directed onto the eye which is to be examined is provided at the actuating device (04), wherein a flow pulse directed onto the eye is produced by increasing the pressure in the pressure chamber (05).

12. Analysis system according to Claim 11,
**characterized in that**
a sensor (09) for direct or indirect measurement of the intensity of the flow pulse is provided in or at the pressure chamber (05).

13. Analysis system according to one of Claims 10 to 12,
**characterized in that**
the ray path of the light slit produced by the slit projector (16), on impinging onto the cornea (03), runs coaxially to the longitudinal axis of the flow pulse of the gaseous medium.

14. Analysis system according to one of Claims 10 to 13,
**characterized in that**
the ray path of the light slit produced by the slit projector (16) runs through the actuating device (04), wherein the actuating device (04) has recesses (06) at the points of passage of the ray path, or is made from transparent material (11).

15. Analysis system according to Claim 14,
**characterized in that**
the ray path of the light slit produced by the slit projector (16) runs through the nozzle opening (06).

16. Analysis system according to one of Claims 10 to 13,
**characterized in that**
deflecting optics are arranged in front of and/or behind the nozzle opening in the pressure chamber, by which the ray path of the slit projector can be guided past the nozzle opening.

17. Analysis system according to one of Claims 1 to 16,
**characterized in that**
the recording device (20) is constructed in the manner of a high-speed recording device, by which several sectional images are recorded as a series of images during the deformation of the cornea (03).

18. Analysis system according to one of Claims 1 to 17,
**characterized in that**
a video sensor is provided in the recording device (20), by which the deformation of the cornea (03) can be observed and recorded, wherein the video sensor reproduces the corresponding image data in the form of a video signal.

19. Analysis system according to Claim 18,
**characterized in that**
the video signal is produced in a digital format or is converted thereto.

20. Analysis system according to Claim 19,
**characterized in that**
the video sensor is constructed in the manner of a CCD chip or a CMOS chip.

21. Analysis system according to Claims 18 to 20,
**characterized in that**
the video sensor is formed by at least one line scan camera.

22. Analysis system according to Claim 21,
**characterized in that**
the video sensor is formed by several line scan cameras arranged parallel to each other and spaced apart from each other.

23. Analysis system according to one of Claims 18 to 20,
**characterized in that**
the video sensor is formed by an area scan camera.

24. Analysis system according to one of Claims 1 to 23,
**characterized in that**
an adjusting camera (10) for correct positional alignment of the eye (02) which is to be examined is provided in the analysis system (01).

## Revendications

1. Système d'analyse ophtalmique (01) pour mesurer la pression intraoculaire à l'intérieur d'un oeil (02), comprenant :
a) un dispositif d'actionnement (04) pour déformer la cornée (03) sans contact,
b) un système d'observation (20, 21) permettant d'observer et d'enregistrer la déformation de la cornée,
c) un dispositif d'analyse (24) par lequel la pression intraoculaire est dérivée à partir des informations d'image du système d'observation (20, 21), ledit système d'observation (20, 21) enregistrant des images en coupe au moins de certaines parties de la cornée (03) non déformée et/ou déformée,
**caractérisé en ce que**
le dispositif d'analyse (24) est aménagé de telle sorte qu'à partir des images en coupe de la cornée (03), la diffusion de la lumière par la cornée (03) est déterminée, dans lequel la diffusion de la lumière par la cornée (03) est dérivée comme une mesure de l'élasticité de la cornée (03), et l'élasticité de la cornée (03), dérivée à partir de la diffusion de la lumière, est prise en compte comme une grandeur d'influence lors de la dérivation de la pression intraoculaire.

2. Système d'analyse selon la revendication 1,
**caractérisé en ce que**
dans le dispositif d'analyse (24), l'épaisseur de la cornée (03) est dérivée à partir des images en coupe de la cornée (03).

3. Système d'analyse selon la revendication 1,
**caractérisé en ce que**
dans le dispositif d'analyse (24), la courbure de la cornée (03) est dérivée à partir des images en coupe de la cornée (03).

4. Système d'analyse selon l'une quelconque des revendications 2 à 3,
**caractérisé en ce que**
lors de la dérivation de la pression intraoculaire, l'épaisseur de la cornée (03) et/ou la courbure de la cornée (03) est/sont prise(s) en compte comme grandeur(s) d'influence.

5. Système d'analyse selon l'une quelconque des revendications 1 à 4,
**caractérisé en ce que**
dans le dispositif d'analyse (24), la pression intraoculaire dans l'oeil (02) est dérivée à partir des images en coupe de la cornée (03) déformée, en particulier à partir d'une rafale d'images en coupe de la cornée (03).

6. Système d'analyse selon l'une quelconque des revendications 1 à 5,
**caractérisé en ce que**
le système d'observation (20, 21) coopère avec un projecteur à fente (16) par lequel une fente de lumière est projetée sur la cornée (03), dans lequel les images en coupe à enregistrer par le système d'observation (20, 21) se trouvent dans un plan d'image éclairé par le projecteur à fente.

7. Système d'analyse selon la revendication 6,
**caractérisé en ce que**
le système d'observation (20, 21) comprend un deuxième dispositif d'enregistrement (20) qui enregistre au moins en partie la cornée (03) dans le plan d'image éclairé par le projecteur à fente (16).

8. Système d'analyse selon la revendication 7,
**caractérisé en ce qu'**
entre la cornée (03) et le dispositif d'enregistrement (20), au moins un objectif (21) est disposé par lequel le plan d'image de la cornée (03), éclairé par le projecteur à fente (16), est reproduit sur un plan d'enregistrement dans le dispositif d'enregistrement (20).

9. Système d'analyse selon la revendication 8,
**caractérisé en ce que**
le plan d'image de la cornée (03), éclairé par le projecteur à fente (16), et le plan d'objectif de l'objectif (21) disposé entre la cornée (03) et le dispositif d'enregistrement (20), et le plan d'enregistrement dans le dispositif d'enregistrement (20) sont disposés sous un angle de telle sorte que le plan d'image de la cornée (03) est reproduit selon la loi de Scheimpflug sur le plan d'enregistrement du dispositif d'enregistrement (20).

10. Système d'analyse selon l'une quelconque des revendications 1 à 9,
**caractérisé en ce qu'**
en vue de déformer la cornée (03), le dispositif d'actionnement (04) applique à la surface de la cornée une impulsion d'écoulement d'un milieu gazeux, en particulier de l'air.

11. Système d'analyse selon la revendication 10,
**caractérisé en ce que**
sur le dispositif d'actionnement (04), une chambre de mise en pression (05) au moins partiellement transparente est prévue avec une ouverture de buse (06) tournée vers l'oeil à examiner, dans lequel une augmentation de la pression dans la chambre de mise en pression (05) génère une impulsion d'écoulement dirigée sur l'oeil.

12. Système d'analyse selon la revendication 11,
**caractérisé en ce que**
dans ou sur la chambre de mise en pression (05), un capteur (09) est prévu pour la mesure directe ou indirecte de l'intensité de l'impulsion d'écoulement.

13. Système d'analyse selon l'une quelconque des revendications 10 à 12,
**caractérisé en ce que**
la trajectoire des rayons de la fente de lumière générée par le projecteur à fente (16) s'étend coaxialement à l'axe longitudinal de l'impulsion d'écoulement du milieu gazeux au moment de l'impact sur la cornée (03).

14. Système d'analyse selon l'une quelconque des revendications 10 à 13,
**caractérisé en ce que**
la trajectoire des rayons de la fente de lumière générée par le projecteur à fente (16) s'étend à travers le dispositif d'actionnement (04), dans lequel le dispositif d'actionnement (04) présente aux points de passage de la trajectoire des rayons des évidements (06) ou est fabriqué à partir d'un matériau transparent (11).

15. Système d'analyse selon la revendication 14,
**caractérisé en ce que**
la trajectoire des rayons de la fente de lumière générée par le projecteur à fente (16) passe par l'ouverture de buse (06).

16. Système d'analyse selon l'une quelconque des revendications 10 à 13,
**caractérisé en ce que**
devant et/ou derrière l'ouverture de buse, dans la chambre de mise en pression, une optique de déviation est disposée, permettant de faire passer la trajectoire des rayons du projecteur à fente devant l'ouverture de buse.

17. Système d'analyse selon l'une quelconque des revendications 1 à 16,
**caractérisé en ce que**
le dispositif d'enregistrement (20) est réalisé à la manière d'un dispositif d'enregistrement rapide par lequel plusieurs images en coupe sont enregistrées sous forme de rafale d'images pendant la déformation de la cornée (03).

18. Système d'analyse selon l'une quelconque des revendications 1 à 17,
**caractérisé en ce que**
dans le dispositif d'enregistrement (20), un capteur vidéo est prévu, permettant d'observer et d'enregistrer la déformation de la cornée (03), dans lequel le capteur vidéo retransmet les données d'image correspondantes sous la forme d'un signal vidéo.

19. Système d'analyse selon la revendication 18,
**caractérisé en ce que**
le signal vidéo est généré ou converti en format numérique.

20. Système d'analyse selon la revendication 19,
**caractérisé en ce que**
le capteur vidéo est réalisé à la manière d'une puce CCD ou d'une puce CMOS.

21. Système d'analyse selon l'une quelconque des revendications 18 à 20,
**caractérisé en ce que**
le capteur vidéo est formé par au moins une caméra à balayage linéaire.

22. Système d'analyse selon la revendication 21,
**caractérisé en ce que**
le capteur vidéo est formé par plusieurs caméras à balayage linéaire, disposées à distance en parallèle les unes aux autres.

23. Système d'analyse selon l'une quelconque des revendications 18 à 20,
**caractérisé en ce que**
le capteur vidéo est formé par une caméra planaire.

24. Système d'analyse selon l'une quelconque des revendications 1 à 23,
**caractérisé en ce que**
dans le système d'analyse (01), une caméra de mise au point (10) est prévue pour aligner correctement l'oeil (02) à examiner.
